# EUROPÄISCHE PATENTSCHRIFT

(11) **EP 0 452 446 B1**
(45) Veröffentlichungstag und Bekanntmachung des Hinweises auf die Patenterteilung: **29.12.1993**
(21) Anmeldenummer: 90915758.8
(22) Anmeldetag: 15.10.1990
(51) Int. Cl.: A61K 7/16

(54) **MUND- UND ZAHNPFLEGEMITTEL**
ORAL AND DENTAL HYGIENE PREPARATION
PREPARATION D'HYGIENE BUCCO-DENTAIRE

(30) Priorität: 14.10.1989 DE 3934416
(43) Veröffentlichungstag der Anmeldung: 23.10.1991
(73) Patentinhaber: Desitin Arzneimittel GmbH, D-22335 Hamburg (DE)
(72) Erfinder: SCHMIDT, Wolfgang, D-2000 Hamburg 63 (DE)
(74) Vertreter: UEXKÜLL & STOLBERG
(86) Internationale Anmeldenummer: EP9001936
(87) Internationale Veröffentlichungsnummer: WO9105540

(56) Entgegenhaltungen:
- EP-A- 0 219 762
- EP-A- 0 259 749
- GB-A- 1 476 057
- GB-A- 2 163 348
- GB-A- 2 186 190

## Beschreibung

Zahnpflegemittel werden seit vielen Jahren als Pasten, sogenannte Zahnpasten hergestellt. Dabei ist der wesentliche Ausgangsstoff eine Schlämmkreide, die mit Wasser, Glycerin, waschaktiven Stoffen und Verdickungsmitteln zu einer Paste verarbeitet und in Tuben oder Spendern abgefüllt wird. Die Zahnpasta hat den Markt erobert, während andere Zahnpflegemittel wie Tropfen, Zahnseifen und - pulver oder Granulate kaum noch eine Rolle spielen. Mit den Mitteln soll der bakterielle Zahnbelag entfernt, Kariesprophylaxe betrieben sowie die Reinigung der Zähne schonend und durch die Bürstenbehandlung wesentlich unterstützt durchgeführt und der Mundraum gründlich gereinigt und angenehm erfrischt werden.

In neuerer Zeit hat sich das Bild der Zahnpasten nicht wesentlich verändert, obwohl die Rezepturen in vielerlei Hinsicht abgewandelt wurden. Die Verwendung einer recht groben Kreideform zum mechanischen Reinigen der Zähne wich mehr und mehr modernen, feineren Poliermitteln auf Basis von Aluminiumoxid oder Siliciumdioxid (Kieselgele). Neben Tensiden finden strukturbildende Komponenten und ausgefeilte Geschmackskorrigentien Verwendung. Oft werden Wirkstoffe wie insbesondere verschiedene Fluorderivate oder Mineralsalze zugefügt. Das Volumen konnte teilweise reduziert werden; sicherlich hat die Einführung und allgemeine Verwendung elektrischer Zahnbürsten hierbei einen starken Einfluß gehabt.

Die Handhabung von Zahnpasten ist jedoch mit einer Reihe von Nachteilen verbunden. Weil die Dosierung aus einfachen Tuben Schwierigkeiten bereitet, hat man in neuerer Zeit Zahnpastaspender entwickelt, welche jeweils eine vorbestimmte Menge Zahnpasta abgeben. Diese Spender sind jedoch verhältnismäßig groß und daher keinesfalls zur Mitnahme auf Reisen geeignet. Tuben sind druckempfindlich und daher auf Reisen ebenfalls nicht ideal. Sowohl in Spendern als auch in Tuben kann Zahnpasta austrocknen, so daß die angebrauchten Behälter dann weggeworfen werden müssen. Ferner lassen sich sowohl Tuben als auch Spender nicht vollständig entleeren. Nach Verbrauch bleiben die aus Metall oder Plastik hergestellten Behälter zurück und verursachen Umweltprobleme.

Aus der GB-A-21 63 348 sind Zahnreinigungstabletten bekannt, welche durch Zerbeißen und längeres Kauen im Munde eine pastenartige Konsistenz annehmen und dann zur Zahnreinigung dienen können. Eine Anwendung in der üblichen Weise durch Aufbringung auf eine Zahnbürste und anschließendes Einführen in den Mund ist nicht möglich. Verbrauchern mit schadhaften Zähnen oder Zahnersatz ist ein Zerbeißen spröder, harter Tabletten nicht möglich. Ferner können Kautabletten dieser Art auch nicht zur Reinigung künstlicher Zähne bzw. Gebisse verwendet werden.

Der Erfindung liegt demgegenüber die Aufgabe zugrunde, eine neue Verabreichungs- und Dosierungsform für Mund- und Zahnpflegemittel zu entwickeln, welche die vorstehend genannten Nachteile nicht aufweist, sich jedoch ähnlich wie Zahnpasta mit Hilfe einer Zahnbürste anwenden läßt.

Insbesondere soll eine genaue Dosierung für eine Zahnreinigung ermöglicht und sichergestellt werden, daß das Mittel vollständig aufgebraucht werden kann, ohne daß Reste in der Packung zurückbleiben.

Das erfindungsgemäße Mund- und Zahnpflegemittel auf Basis von Tensiden, Poliermitteln, Aromastoffen sowie weiteren üblichen Zusätzen ist dadurch gekennzeichnet, daß die Wirk- und Zusatzstoffe in ein Bindemittel oder eine Bindemittelmischung eingearbeitet sind, welche aus wasserlöslichen oder -quellbaren, physiologisch unbedenklichen Folienbildnern bestehen, wobei die gebildete Folie in Dosiseinheiten vorzerteilt ist.

Als Bestandteile des Mund- und Zahnpflegemittels kommen die Komponenten in Frage, welche üblicherweise zur Herstellung von Zahnpasten Verwendung finden, wobei natürliche Rohstoffe besonders bevorzugt sind. Wichtig ist darüber hinaus, daß alle Bestandteile völlig ungiftig und physiologisch unbedenklich sind, was selbstverständlich auch für die verwendeten Folienbildner gilt. Als wesentliche Bestandteile von Zahnpflegemitteln sind zu nennen:
- Schleifmittel wie Kreide (Calciumcarbonat), Calcium- und Natriumphosphate, Aluminiumoxid oder Siliciumdioxid, insbesondere Kieselgele
- Tenside (Schaummittel) wie Natriumlaurylsulfat, Natriumlaurylsulfoacetat, Sarcoside, Monoglyceridsulfate und andere
- Aromastoffe wie Pfefferminzöl, Krauseminzöl, Anisöl, Zimtöl, Nelkenöl, Menthol und ähnliche
- Süßstoffe wie Saccharin, Cyclamat, Aspartam und ähnliche.

Die in Zahnpasten üblicherweise enthaltenen flüssigen Komponenten wie Glycerin, Propylenglykol oder Sorbitsirup müssen den erfindungsgemäßen Mitteln in Folienform nicht in den üblichen Mengen zugesetzt werden, da hier die für Tuben oder Spender erforderliche Plastizität keine Rolle spielt. Weitere übliche Zusätze wie Fluorverbindungen, Mittel gegen Zahnsteinbildung, antibakterielle Wirkstoffe und ähnliche, wie sie in Mund- und Zahnpflegemitteln üblicherweise Verwendung finden, können auch erfindungsgemäß eingesetzt werden.

Als wasserlösliche bzw. -quellbare Folienbildner eignen sich vor allem Stärken, Gelatinen, Glycerin und/oder Sorbit sowie ferner natürliche oder synthetische Harze und Gumme. Folgende Rahmenrezeptur hat sich bewährt:
- Gelatine: 8 - 10 g
- Stärke: 3 - 8 g
- Glycerin: 1 - 2 g
- Wasser: 30 - 50 g.

In dieser Grundmasse werden die Bestandteile des Mund- und Zahnpflegemittels gelöst bzw. dispergiert, um eine gleichmäßige Verteilung der Stoffe zu erreichen. Die so erhaltene Mischung kann erfindungsgemäß in verschiedener Weise zu einem folienförmigen Mund- und Zahnpflegemittel verarbeitet werden:
a) Es ist einmal möglich, die Masse direkt zu einer Folie zu verarbeiten, welche im allgemeinen eine Dicke zwischen 0,1 und etwa 3 mm aufweist. Durch Sollbruchstellen mittels Stanzung oder Perforierung kann diese Folie in Dosiseinheiten vorzerteilt werden, wobei die Streifenbreite und -länge vorzugsweise etwa der Zahnbürstengröße, d.h. der von den freien Borstenenden gebildeten Fläche des Borstenblocks oder der Längsquerschnittfläche des Borstenblocks in der Borstenebene entsprechen sollte.
b) Alternativ kann die Masse auf eine Trägerfolie aufgebracht werden, deren Zusammensetzung derjenigen des Bindemittels der Masse entspricht, wie dies in der EP-A-219,762 im einzelnen offenbart ist. Auch die auf diese Weise erhaltenen Folien können wie oben angegeben vorzerteilt werden.
c) Es ist ferner möglich, die Masse auf eine Releasefolie oder ein Releasepapier aufzubringen, wie dies aus der EP-A-259 749 bekannt ist. In diesem Fall wird die Beschichtung in einzelne Abschnitte der oben angegebenen Größe vorzerteilt, welche sich ähnlich wie Haftetiketten von der Trägerfolie vor Gebrauch abziehen lassen.

In allen Fällen erhält man eine Darreichungs- und Dosierungsform, deren Anwendung besonders leicht ist, da die jeweils zu verwendende Menge gleichmäßig vorgegeben ist. Eine Dosis wird in Form eines Folienabschnittes abgetrennt bzw. abgezogen und auf die angefeuchtete Zahnbürste bzw. zwischen die Borsten gelegt, wo sie durch die Feuchtigkeitsberührung haftet und anquillt. Durch das Einführen in die Mundhöhle und in Verbindung mit dem Speichel und der intensiven Zahnbürstenbewegung wird der Streifen an- und aufgelöst, so daß die Inhaltsstoffe zur vollen Wirkung gelangen. Nach der Anwendung und der anschließenden Mundspülung mit Wasser verbleiben keinerlei Rückstände im Mund.

Gewünschtenfalls können die Folien in unterschiedlicher Weise bedruckt, geprägt oder gestanzt werden, wobei beispielsweise für Kinder auch bildliche Darstellungen möglich sind. Es entfällt das Öffnen und Schließen von Tubenverschlüssen, es wird keine Zahnpasta vergeudet und die erfindungsgemäße Darreichungsform läßt sich auch besondes gut auf Reisen einsetzen, da sie leicht ist, ein Auslaufen nicht befürchtet werden muß und sie äußerst wenig Platz beansprucht. Die Verpackung ist umweltfreundlich in Pappschachteln ohne Verwendung von Metallen oder Kunststoff möglich.

Die Mittel der Erfindung eignen sich nicht nur zur Zahnpflege im Mund, sondern bei geeigneter Zusammensetzung auch zur Reinigung und Pflege von künstlichen Zähnen und Gebissen. Für diesen letzteren Einsatzzweck ist eine Mehrfachbeschichtung besonders günstig, bei der sich in einer Schicht die reinigenden, desinfizierenden und sauren Komponenten befinden, während sich, ggf. getrennt durch eine ebenfalls wasserlösliche Sperrschicht, in einer zweiten Schicht die CO₂ bzw. O₂ abgebenden Substanzen enthalten sind.

### Beispiel

Ein erfindungsgemäßes Zahnpflegemittel hat folgende Zusammensetzung:
- Amylogum: 57,0 g
- Honig: 25,0 g
- Zitronensäure: 2,0 g
- Titandioxid: 1,0 g
- Aroma: 1,0 g
- Siliciumdioxid: 3,0 g
- Ca-Hydrog-phos.: 10,0 g
- Na-Laurylsulfat: 1,0 g

Mit der erforderlichen Menge Wasser wird ein Brei hergestellt, der zu einer Folie verarbeitet wird, die ca. 0,5 mm dick ist. Durch Perforation wird die Folie in Abschnitte von 8 x 35 mm unterteilt.

Gegebenenfalls kann die Masse auch als Beschichtung auf ein Releasepapier als Träger aufgebracht und durch Stanzung in Abschnitte der angegebenen Größe vorzerteilt werden.

## Patentansprüche

1. Mund- und Zahnpflegemittel auf Basis von Tensiden, Poliermitteln, Aromastoffen sowie weiteren üblichen Zusatzstoffen, dadurch gekennzeichnet, daß die Wirk- und Zusatzstoffe in ein Bindemittel oder eine Bindemittel-Mischung eingearbeitet sind, welche aus wasserlöslichen oder -quellbaren, physiologisch unbedenklichen Folienbildnern bestehen, und daß diese Mischung zu einer Folie verarbeitet ist, wobei die so gebildete Folie in Dosiseinheiten vorzerteilt ist.

2. Mund- und Zahnpflegemittel gemäß Anspruch 1, dadurch gekennzeichnet, daß es als Folienbildner Stärken, Gelatinen, Glycerin und/oder Sorbitol oder natürliche und/oder synthetische Harze und Gumme enthält.

3. Mund- und Zahnpflegemittel gemäß Anspruch 1, dadurch gekennzeichnet, daß es als Folienbildner Amylogum enthält.

4. Mund- und Zahnpflegemittel nach den Ansprüchen 1 bis 3, dadurch gekennzeichnet, daß es als Folienbildner eine Mischung aus 8 bis 10 Gewichtsteilen Gelatine, 4 bis 8 Gewichtsteilen Stärke und 1 bis 2 Gewichtsteilen Glycerin enthält.

5. Mund- und Zahnpflegemittel nach den Ansprüchen 1 bis 4, dadurch gekennzeichnet, daß es aus einer Trägerfolie aus dem Bindemittel oder der Bindemittel-Mischung besteht, auf welche eine Schicht aufgebracht ist, welche die Bestandteile des Pflegemittels zusammen mit Bindemittel oder der Bindemittel-Mischung enthält, wobei das Bindemittel oder die Bindemittel-Mischung in der Trägerfolie und in der Beschichtung im wesentlichen die gleiche qualitative Zusammensetzung aufweisen.

6. Mund- und Zahnpflegemittel nach den Ansprüchen 1 bis 4, dadurch gekennzeichnet, daß eine Beschichtung aus den Bestandteilen des Pflegemittels und dem Bindemittel oder der Bindemittel-Mischung auf eine Trägerfolie in Form eines Trennpapiers, eines Trennfilms oder einer Trennfolie aufgebracht ist, wobei die Beschichtung nach Vorzerteilung in Dosiseinheiten von dem Trägermaterial dosisweise abziehbar ist.

## Claims

1. Oral and dental hygiene preparation based on surfactants, polishing agents, flavours, and other conventional additives, characterised in that the active ingredients and additives are incorporated in a binder or a binder mixture comprising water-soluble or water-swellable, physiologically harmless film formers, and in that said mixture is processed to a film, the film thus formed being predivided into dose units.

2. Oral and dental hygiene preparation according to claim 1, characterised in that it contains as film formers starches, gelatins, glycerol and/or sorbitol or natural and/or synthetic resins and gums.

3. Oral and dental hygiene preparation according to claim 1, characterised in that it contains starch gum as film former.

4. Oral and dental hygiene preparation according to claims 1 to 3, characterised in that it contains as film former a mixture of 8 to 10 parts by weight of gelatin, 4 to 8 parts by weight of starch and 1 to 2 parts by weight of glycerol.

5. Oral and dental hygiene preparation according to claims 1 to 4, characterised in that it comprises a carrier film made of the binder or the binder mixture, onto which is deposited a layer which contains the constituents of the hygiene preparation together with binder or the binder mixture, whereby the binder or the binder mixture in the carrier film and in the coating have essentially the same qualitative composition.

6. Oral and dental hygiene preparation according to claims 1 to 4, characterised in that a coating consisting of the constituents of the hygiene preparation and the binder or the binder mixture is deposited on a carrier film in the form of a release paper, a release film or a release sheet, whereby the coating can be removed in doses from the carrier material after predivision into dose units.

## Revendications

1. Préparation d'hygiène bucco-dentaire à base d'agents tensio-actifs, d'agents de polissage, de substances aromatiques ainsi que d'autres ingrédients habituels, caractérisée en ce que les principes actifs et les ingrédients additionnels sont incorporés à un agent liant ou à un mélange d'agents liants, qui sont constitués d'agents filmogènes solubles ou gonflables dans l'eau, physiologiquement sans danger, le film formé étant prédivisé en unités de dosage.

2. Préparation d'hygiène bucco-dentaire selon la revendication 1, caractérisée en ce qu'elle contient à titre d'agents filmogènes des amidons, des gélatines, de la glycérine et/ou du sorbitol ou des résines et des gommes naturelles et/ou synthétiques.

3. Préparation d'hygiène bucco-dentaire selon la revendication 1, caractérisée en ce qu'elle contient à titre d'agent filmogène de l'amylogum.

4. Préparation d'hygiène bucco-dentaire selon les revendications 1 à 3, caractérisée en ce qu'elle contient à titre d'agent filmogène un mélange de 8 à 10 parties en poids de gélatine, de 4 à 8 parties en poids d'amidon et de 1 à 2 parties en poids de glycérine.

5. Préparation d'hygiène bucco-dentaire selon les revendications 1 à 4, caractérisée en ce qu'elle est constituée d'une feuille de support formée de l'agent liant ou du mélange d'agents liants, feuille de support sur laquelle est appliquée une couche qui contient les composants de la préparation d'hygiène conjointement avec l'agent liant ou le mélange d'agents liants, l'agent liant ou le mélange d'agents liants de la feuille de support et du revêtement ayant essentiellement la même composition qualitative.

6. Préparation d'hygiène bucco-dentaire selon les revendications 1 à 4, caractérisée en ce que l'on applique un revêtement formé des composants de la préparation d'hygiène et de l'agent liant ou du mélange d'agents liants sur une feuille de support sous la forme d'un papier de séparation, d'un film de séparation ou d'une feuille de séparation, le revêtement pouvant être séparé de la matière de support par doses individuelles après prédivision en unités de dosage.
